(19) Europäisches Patentamt
European Patent Office
Office européen des brevets

(11) **EP 4 776 294 A2**

(12) **EUROPEAN PATENT APPLICATION**

(43) Date of publication:
**15.07.2026 Bulletin 2026/29**

(21) Application number: **26181594.8**

(22) Date of filing: **21.04.2021**

(51) International Patent Classification (IPC):
**G16C 20/70** (2019.01)

(52) Cooperative Patent Classification (CPC):
**G01N 30/8662; B01D 15/08; C07K 1/16;
G01N 30/8693; G16B 40/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.04.2020 US 202063014273 P**

(62) Document number(s) of the earlier application(s) in
accordance with Art. 76 EPC:
**21724482.1 / 4 139 325**

(71) Applicant: **Amgen Inc.
Thousand Oaks, CA 91320-1799 (US)**

(72) Inventor: **The designation of the inventor has not
yet been filed**

(74) Representative: **Murphy, Orla et al
Kilburn & Strode LLP
Lacon House
84 Theobalds Road
London WC1X 8NL (GB)**

Remarks:
This application was filed on 28-05-2026 as a
divisional application to the application mentioned
under INID code 62.

(54) **SELECTING CHROMATOGRAPHY PARAMETERS FOR MANUFACTURING THERAPEUTIC PROTEINS**

(57)    In a method for facilitating selection of chromatography parameters for manufacturing a therapeutic protein, one or more process parameter values associated with a hypothetical chromatography process, and one or more molecular descriptors descriptive of the therapeutic protein, are received. The method also includes predicting a performance indicator for the hypothetical chromatography process at least by analyzing the one or more process parameters and the one or more molecular descriptors using a machine learning model. The machine learning model is a regression tree model, an eXtreme gradient boost model, or an elastic net model. The method also includes causing the predicted performance indicator, and/or an indication of whether the predicted performance indicator satisfies one or more acceptability criteria, to be presented to a user via a user interface.

**EP 4 776 294 A2**

**Description**

**FIELD OF DISCLOSURE**

The present application relates generally to the production of biopharmaceutical products, and more specifically relates to techniques for modeling a chromatography process (such as a chromatography purification process) to facilitate the selection of chromatography parameters when manufacturing therapeutic proteins.

**BACKGROUND**

In the biopharmaceutical industry, large, complex protein molecules known as biologics or therapeutic proteins are derived from living systems. At a high level, the process of manufacturing the therapeutic proteins includes the following steps: (1) a host cell selection stage, in which the master cell line containing the gene that makes the desired protein is produced (e.g., using Chinese hamster ovary (CHO) cells); (2) a cell culture stage, in which defined culture media are used to grow large numbers of cells that produce the protein in bioreactors; (3) a purification stage, in which the recovery and purification of the product from the previous stage is performed to isolate the protein; and (4) a formulation and fill-finish-package stage, in which the protein is prepared for use by physicians or patients.

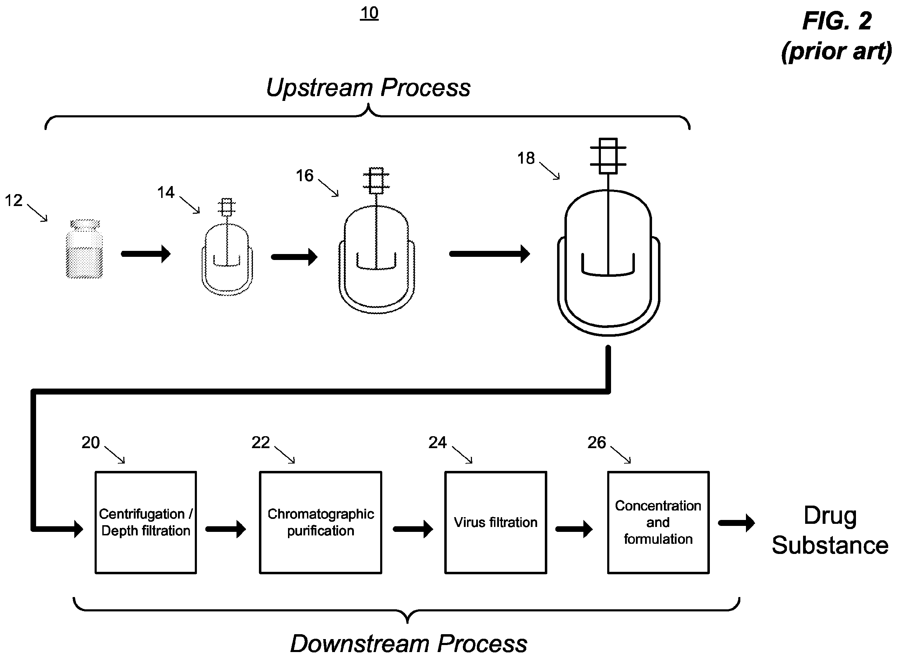

FIG. 2 depicts a typical therapeutic protein manufacturing process 10. At a first stage 12, after an optimal cell that produces high concentrations of the desired protein is engineered and cryopreserved in vials or cell bags, an "upstream" manufacturing process is initiated by reviving the cryopreserved cells. The cells are typically thawed into small T-flasks, shake flasks, or spinner flasks, and expanded in increasing numbers and increasing flask sizes to achieve inoculation of a seed bioreactor at stage 14. Throughout the expansion process, the cells are kept in controlled conditions (e.g., temperature, pH, and/or nutrients) for continued growth. Following one or more stages of culture volume expansion (denoted as stage 16 in FIG. 2), the cells are inoculated into a production bioreactor at stage 18. During stage 18, the therapeutic protein is expressed by the cells. Following this step, a "downstream" process begins. In the downstream process, centrifugation or depth filtration is performed at stage 20 to separate the culture medium from cells and/or separate the desired protein from other molecules in the bioreactor. At stage 22, a chromatographic purification process further isolates the desired protein from the host cells and impurities or other undesired matter (e.g., degraded or aggregated proteins, etc.). Various filtering technologies may be used at stage 22 to isolate and purify the proteins based on their size, molecular weight, and electrical charge. The resulting substance undergoes virus filtration at stage 24. The purified protein is typically formulated with an excipient to produce a sterile solution that can be injected or infused. At stage 26, the substance is concentrated and placed in a target buffer, yielding a formulation which is placed within containers (e.g., vials or syringes) for labeling, long-term storage, and shipment. This example therapeutic manufacturing process 10 is provided for illustrative purposes, but it will be appreciated that the selection of chromatography parameters described herein can readily be applied to other therapeutic protein manufacturing processes that include chromatography.

In general, "chromatography" (e.g., as performed at stage 22) refers to a separation process wherein molecules are distributed between two phases: (1) a stationary phase, which is often a chromatography resin; and (2) a mobile phase, which in the case of protein separation is a solvent, such as water or chloroform. Molecules that are more strongly attracted to the stationary phase move more slowly through the system as compared to those that are more strongly attracted to the mobile phase. For commercial manufacturing purification, chromatography is typically carried out as column chromatography due to scale considerations. In a common chromatographic operation, a volume of sample is injected into the column. Eluent is then pumped through the column, causing molecules to be separated based on their relative affinity for the stationary resin and the eluent. Different molecules will elute from the column at different times and after different volumes of eluent have passed through the column. Accordingly, therapeutic proteins can be separated from other substances that elute from the column at different times. This information is captured in a chromatogram, which is a plot of the concentration exiting the column versus time.

Hydrophobic interaction chromatography can be used to separate proteins based on differences in their hydrophobicity, affinity chromatography can be used to separate molecules based on differences in their affinity for a target ligand attached to a chromatography resin, and ion exchange chromatography can be used to separate molecules based on differences in molecular charge. As a more specific example, cation-exchange chromatography (CEX) is an ion exchange chromatography used when the molecule of interest is positively charged. Proteins have amino acids with acidic and basic side chains. Depending on the acidity level (pH) of the solution surrounding the biologic, the molecule can be positively charged, negatively charged, or neutral. The isoelectric point (pI) is the pH at which the number of protonated and deprotonated groups is equal, and the protein has no net charge. If the pH is greater than the pI, a protein will have a net negative charge, and if the pH is less than the pI, the protein will have a net positive charge. Because the pI of a protein is determined by the primary amino acid sequence of the protein, and can thus be calculated, a buffer can be chosen that ensures a known net charge for a protein of interest. Proteins with different pI values will have varying degrees of charge at a given pH, and so different proteins will bind to the resin with different strengths, facilitating their separation through the column. Other common types of chromatography include size-exclusion chromatography (SEC), in which molecules in solution are separated by size and/or molecular weight, and Protein A chromatography.

Conventionally, selecting chromatography parameters (e.g., elution buffer pH, elution buffer conductivity, elution buffer molarity, gradient slope, linear velocity, load, and collection times), and determining how the purification stage will perform for a particular product/molecule (e.g., with a particular solution, at a particular pH, etc.) can be highly resource-intensive in terms of time, cost, labor, and usage of equipment, and can require a great deal of trial and error by setting up and running numerous experiments to obtain empirical measurements. As the pace of biotechnology quickens, however, and as an increased emphasis is placed on processing additional molecules in the pipeline, there is an increasing need to more quickly design and implement manufacturing processes, including the chromatographic purification process.

## SUMMARY

Embodiments described herein relate to systems and methods that create and apply one or more models that are predictive of performance of a purification process in the manufacture of therapeutic proteins. The therapeutic proteins may be any suitable type of protein, such as a monoclonal antibody ("mAb") or a bispecific or other multi-specific antibody, for example. More specifically, in these embodiments, machine learning models are used to predict performance indicators (e.g., product yield and/or quality metrics) of a chromatography purification process, such as a CEX, SEC, Protein A, or any other suitable chromatography process, based on various process parameters (e.g., buffer and/or elution buffer and/or load pH, elution buffer molarity, elution buffer conductivity, gradient slope, linear velocity, load conductivity, load factor, stop collect, column volume, actual CEX loading (if CEX is used), loading flow rate, elution flow rate, buffer concentration, gradient length, gradient start, gradient end, pool volume, protein concentration, pool start, and/or pool end) and molecular descriptors (e.g., mathematical representations of physical characteristics of a molecule). The process may result in a better selection of chromatography process parameters as compared to conventional processes, a substantial reduction in the amount of time required to design/develop/implement the downstream manufacture process (e.g., by obviating or reducing the need to conduct experiments), and/or a substantial reduction in the usage of other resources (e.g., labor, equipment, costs). Moreover, the process may reveal how different molecule physical characteristics (relating to various molecule descriptors) affect chromatography performance, thereby providing insights into molecule design.

It is contemplated that performance indicators are typically dependent on process parameters. As described herein, non-null process parameters can be predicted based on one or more performance indicator, permitting the efficient prediction of process parameters (or accuracy ranges thereof) based on one or more desired process parameters. Some embodiments herein relate to systems and methods that create and apply one or more models that facilitate selection of chromatography parameters for a purification process based on one or more desired performance indicators. These methods may be used for facilitating selection of chromatography parameters for a purification process during manufacture of a therapeutic protein. In these embodiments, machine learning models are used to predict process parameters (e.g., buffer and/or elution buffer and/or load pH, elution buffer molarity, elution buffer conductivity, gradient slope, linear velocity, load conductivity, load factor, stop collect, column volume, actual CEX loading (if CEX is used), loading flow rate, elution flow rate, buffer concentration, gradient length, gradient start, gradient end, pool volume, protein concentration, pool start, and/or pool end) based on one or more performance indicator (e.g., product yield and/or quality metrics) of a chromatography purification process, such as a CEX, SEC, Protein A, or any other suitable chromatography process, based on various process parameters and molecular descriptors (e.g., mathematical representations of physical characteristics of a molecule).

Moreover, interpretable machine learning algorithms may be used to identify the input features (e.g., molecular descriptors and process parameters or performance indicators) that are most important to generating accurate predictions. This can be particularly helpful given that the number of process parameters, performance indicators, and especially the number of potential molecular descriptors, can be vast (e.g., hundreds or even thousands of potential molecular descriptors). Thus, for example, it may be possible to make sufficiently accurate predictions for the purification process using a relatively small number of input features, and eliminating the need to measure or calculate numerous other parameters and/or descriptors. Knowledge of the correlations between input parameters/descriptors and prediction targets can also provide scientific insight, and spawn hypotheses for further investigation that can lead to future bioprocess improvements.

## BRIEF DESCRIPTION OF THE DRAWINGS

The skilled artisan will understand that the figures, described herein, are included for purposes of illustration and do not limit the present disclosure. The drawings are not necessarily to scale, and emphasis is instead placed upon illustrating the principles of the present disclosure. It is to be understood that, in some instances, various aspects of the described implementations may be shown exaggerated or enlarged to facilitate an understanding of the described implementations. In the drawings, like reference characters throughout the various drawings generally refer to functionally similar and/or structurally similar components.

FIG. 1 is a simplified block diagram of an example system that may implement the techniques described herein.

FIG. 2 depicts a prior art process for manufacturing a drug substance.

FIG. 3 is a flow diagram of an example process for generating a machine learning model for use in the system of FIG. 1.

FIGs. 4A and 4B depict example feature importance metrics for predicting experimental yield or SE-HPLC HMW using an eXtreme gradient boost model.

FIGs. 5A and 5B are flow diagrams of example methods for facilitating selection of chromatography parameters for a purification process during the manufacture of therapeutic proteins.

## DETAILED DESCRIPTION

The various concepts introduced above and discussed in greater detail below may be implemented in any of numerous ways, and the described concepts are not limited to any particular manner of implementation. Examples of implementations are provided for illustrative purposes.

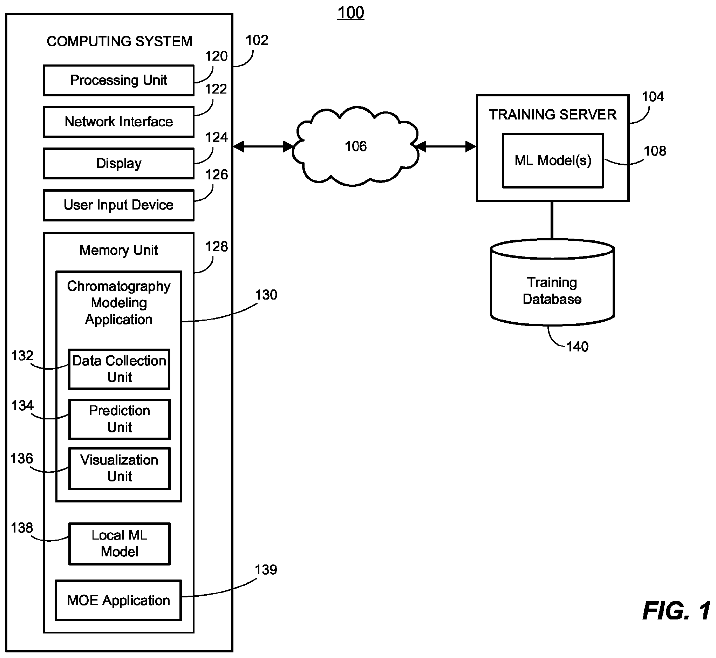

FIG. 1 is a simplified block diagram of an example system 100 that may implement the techniques described herein. System 100 includes a computing system 102 communicatively coupled to a training server 104 via a network 106. Generally, computing system 102 and/or training server 104 are configured to train one or more machine learning (ML) models 108, and use the trained model(s) to predict performance (e.g., yield and/or product quality metrics) for hypothetical chromatography processes that can be used in the manufacture of therapeutic proteins. It should be appreciated that the term "hypothetical," as used herein, does not necessarily mean that no corresponding, real-world process exists. For example, predicted performance may be compared with measured performance by running one of ML model(s) 108 in parallel with, or even after, a corresponding real-world chromatography purification process. The chromatography purification process may include at least one of a CEX process, an SEC process, a Protein A chromatography process, or any other suitable chromatography process.

The ML model(s) 108 may predict performance based on process parameters (e.g., elution buffer pH, salt concentration, column volume, etc.), molecular descriptors (e.g., parameters including or relating to molecule charge, hydrophobicity, isoelectric point, dipole moment, etc.), and/or other numerical and/or categorical parameters (e.g., modality, such as monoclonal antibody (mAb)) or bispecific antibody, etc.). Computing system 102 is also generally configured to enable one or more users, who may be local or remotely distributed, to make use of the prediction capabilities of computing system 102, and to provide various interactive capabilities to the user(s) as discussed elsewhere herein.

Network 106 may be a single communication network, or may include multiple communication networks of one or more types (e.g., one or more wired and/or wireless local area networks (LANs), and/or one or more wired and/or wireless wide area networks (WANs) such as the Internet). In various embodiments, training server 104 may train and/or utilize ML model(s) 108 as a "cloud" service (e.g., Amazon Web Services), or training server 104 may be a local server. In the depicted embodiment, however, ML model(s) 108 is/are trained by server 104, and then transferred to computing system 102 via network 106 as needed. In other embodiments, one, some or all of ML model(s) 108 may be trained on computing system 102, and then uploaded to server 104. In still other embodiments, computing system 102 trains and maintains/-stores the model(s) 108, in which case system 100 may omit both network 106 and training server 104, or server 104 may be a part of computing system 102.

Computing system 102 may include one or more general-purpose computers specifically programmed to perform the operations discussed herein, and/or may include one or more special-purpose computing devices. As seen in FIG. 1, computing system 102 includes a processing unit 120, a network interface 122, a display 124, a user input device 126, and a memory unit 128. In embodiments where computing system 102 includes two or more computers (either co-located or remote from each other), the operations described herein relating to at least processing unit 120, network interface 122, and/or memory unit 128 may be divided among multiple processing units, multiple network interfaces, and/or multiple memory units, respectively. Moreover, display 124 and user input device 126, while referred to herein in the singular, may include multiple displays and multiple user input devices, respectively. For example, display 124 may include at least one display at each of a number of remote, user-specific client devices, and user input device 126 may include at least one user input device for each of those client devices.

Processing unit 120 includes one or more processors, each of which may be a programmable microprocessor that executes software instructions stored in memory unit 128 to execute some or all of the functions of computing system 102 as described herein. Processing unit 120 may include one or more central processing units (CPUs) and/or one or more graphics processing units (GPUs), for example. Alternatively, or in addition, some of the processors in processing unit 120 may be other types of processors (e.g., application-specific integrated circuits (ASICs), field-programmable gate arrays (FPGAs), etc.), and some of the functionality of computing system 102 as described herein may instead be implemented in hardware.

Network interface 122 may include any suitable hardware (e.g., a front-end transmitter and receiver hardware), firmware, and/or software configured to communicate with training server 104 via network 106 using one or more communication protocols. For example, network interface 122 may be or include an Ethernet interface, enabling computing system 102 to communicate with training server 104 over the Internet or an intranet, etc.

Display 124 may use any suitable display technology (e.g., LED, OLED, LCD, etc.) to present information to a user, and user input device 126 may be a keyboard or other suitable input device. In some embodiments, display 124 and user input device 126 are integrated within a single device (e.g., a touchscreen display). Generally, display 124 and user input device 126 may combine to enable a user to interact with graphical user interfaces (GUIs) provided by computing system 102. However, computing system 102 may omit display 124 and/or user input device 126, e.g., in certain embodiments where computing system 102 interacts with other computing devices or systems (e.g., client devices of third parties) to enable interaction by users of those devices or systems.

Memory unit 128 may include one or more volatile and/or non-volatile memories. Any suitable memory type or types may be included, such as read-only memory (ROM), random access memory (RAM), flash memory, a solid-state drive (SSD), a hard disk drive (HDD), and so on. Collectively, memory unit 128 may store one or more software applications, the data received/used by those applications, and the data output/generated by those applications. These applications include a chromatography modeling application 130 that, when executed by processing unit 120, predicts performance (e.g., yield and/or quality metrics) of a hypothetical chromatography process for purification during therapeutic protein manufacture. In some embodiments, the various "units" of application 130 discussed herein may be distributed among different software applications, and/or the functionality of any one such unit may be divided among two or more software applications.

In the example system 100, application 130 includes a data collection unit 132, a prediction unit 134, and a visualization unit 136. In general, data collection unit 132 receives (e.g., retrieves) the parameters that prediction unit 134 applies as inputs to a local machine learning (ML) model 138, to predict a performance indicator (e.g., a yield or product quality metric) or a process parameter (or accuracy range thereof). In the depicted embodiment, ML model 138 is a local copy of one of the model(s) 108 trained by training server 104, and may be stored in a RAM of memory unit 128, for example. As noted above, however, server 104 may utilize/run all models 108 in some embodiments, in which case no local copy need be present in memory unit 128, or all of model(s) 108 may reside in a persistent memory of memory unit 128 rather than being retrieved from training server 104 on an as-needed basis. Data collection unit 132 may receive the values from a user entering parameters/values via a GUI (e.g., on display 124) that is generated or populated by visualization unit 136, and/or may receive values as one or more files or other data transfers (e.g., using file paths designated by a user via such a GUI), for example.

Visualization unit 136 may also generate and/or populate a GUI to view and/or interact with the predicted results of that modeled process (e.g., values of the performance indicator output or process parameter output by prediction unit 134 using model 138), for example. Depending on the embodiment, visualization unit 136 may also provide tools for users to develop useful models (e.g., identify the most predictive features for a given performance indicator or process parameter, optimize hyperparameters for the model, etc.), and/or for users to make use of such models when designing (e.g., optimizing) a chromatography purification process (e.g., to achieve a process with high yield and good product quality attributes, and/or a process that is highly consistent/repeatable, etc.).

In the example system 100, memory unit 128 also stores software instructions of a molecular operating environment (MOE) application 139 that provides homology modeling for therapeutic proteins of interest. Generally, MOE application 139 is configured to generate descriptors for a molecule (e.g., mathematical representations of physical characteristics of the molecule, such as charge, hydrophobicity, dipole moment, isoelectric point, etc.) based on input information about the molecule. For example, a user may use MOE application 139 to enter the amino acid sequence of the molecule (e.g., via user input device 126), and to select an appropriate molecule template. MOE application 139 may then attempt to "fit" the amino acid sequence to the selected template. Alternatively, or in addition, MOE application 139 may generate the descriptors based on experimental/measured results for the molecule. In alternative embodiments, MOE application 139 is stored and executed by a computing device or system other than computing system 102 (e.g., by a third party computing device or system).

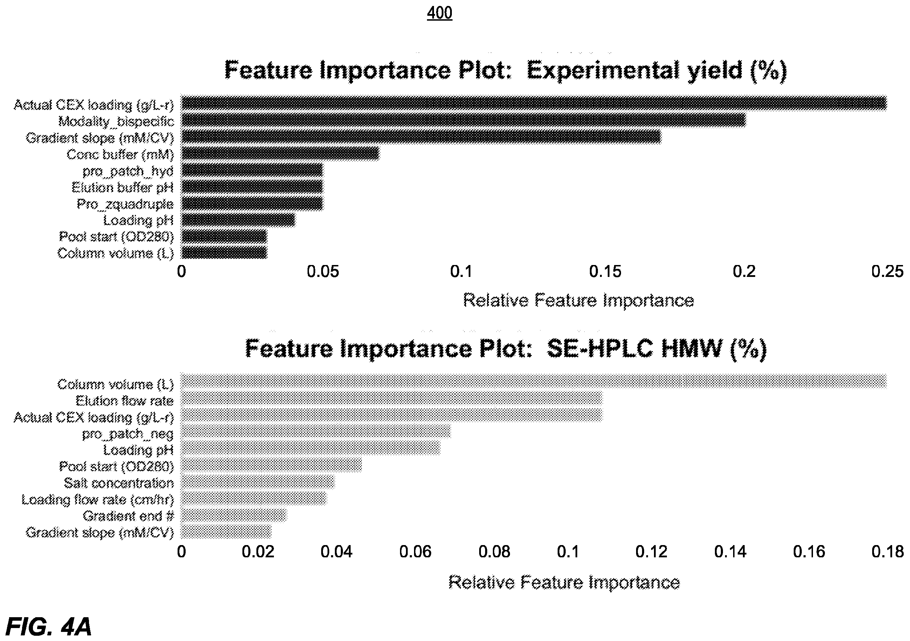

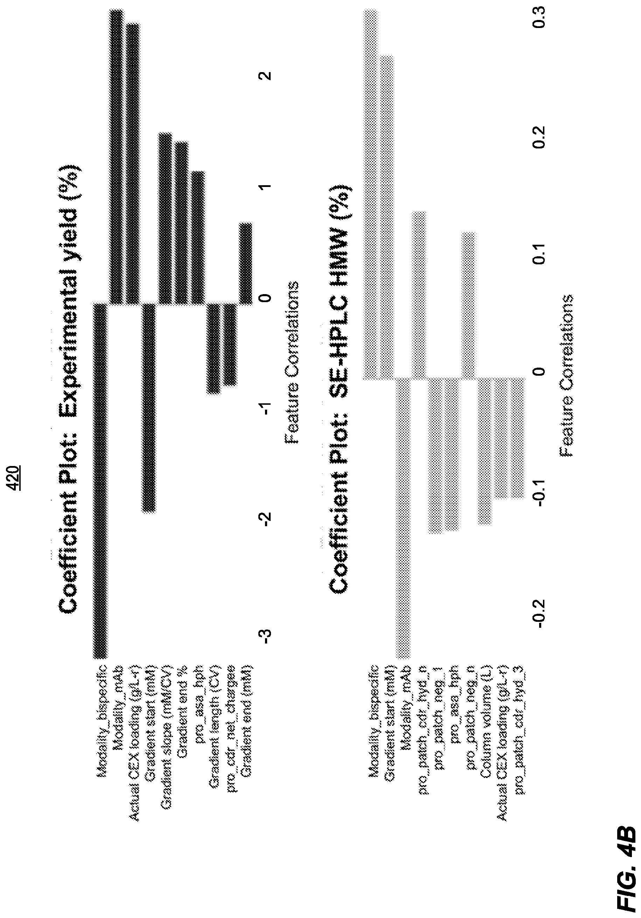

Operation of system 100, according to one embodiment, will now be described in further detail. Initially, training server 104 trains ML model(s) 108 using historical data stored in a training database 140. Training database 140 may include a single database stored in a single memory (e.g., HDD, SSD, etc.), or may include multiple databases stored in one or more memories. ML model(s) 108 may include a number of different types of machine learning models, such as an eXtreme gradient boost (or "xgboost") model, a regression (or "decision" or "ID") tree model, an elastic net model, a lasso model, a ridge model, a stochastic gradient descent (SGD) regularized loss linear model, a linear support vector machine (SVM) model, a partial least squares (PLS) regression model, and/or one or more other suitable model types. Moreover, different models of ML models 108 may be trained to predict different performance indicators (e.g., yield, specific CEX readings, specific SEC readings, etc.) or different process parameters (e.g., buffer and/or elution buffer and/or load pH, elution buffer molarity, elution buffer conductivity, gradient slope, linear velocity, load conductivity, load factor, stop collect, column volume, actual CEX loading (if CEX is used), loading flow rate, elution flow rate, buffer concentration, gradient length, gradient start, gradient end, pool volume, protein concentration, pool start, and/or pool end). In some embodiments, for example, ML models 108 specifically include a regression tree model for predicting a first set of one or more performance indicators, an xgboost model for predicting a different, second set of one or more performance indicators, and an elastic net model for predicting a different, third set of one or more performance indicators. In some embodiments, for example, ML models 108 specifically include a regression tree model for predicting a first set of one or more process parameters, an xgboost model for predicting a different, second set of one or more process parameters, and an elastic net model for predicting a different, third set of one or more process parameters. Further, in some embodiments, ML model(s) 108 may include more than one model of any given type (e.g., two or more models of the same type that are trained on different historical datasets, using different feature sets, and/or having different hyperparameters). In some embodiments, and as discussed in further detail in connection with FIGs. 4A and 4B, each of ML model(s) 108 may be used to identify which features (e.g., process parameters, molecular descriptors, etc.) are most predictive of a particular performance indicator (as applicable), which features (e.g., performance indicators, molecular descriptors, etc.) are most predictive of a process parameter (as applicable), and/or may be trained or re-trained using a feature set that only includes the features that are most predictive of a particular performance indicator or process parameter.

For each different model within ML model(s) 108, training database 140 may store a corresponding set of training data (e.g., input/feature data, and corresponding labels), with possible overlap between the training data sets. To train a model that predicts yield percentage, for instance, training database 140 may include numerous sets of inputs/features each comprising historical process parameters (e.g., pH levels, loading flow rates, salt concentrations, etc.), which may have been made by analytical instruments, as well as molecular descriptors (e.g., descriptors relating to charge, hydrophobicity, isoelectric point, etc.) calculated by software (e.g., by MOE application 139 or similar software) for the protein that was being manufactured, and possibly other information (e.g., modality of the protein that was being manufactured), along with a label for each feature set. In this example, the label for each feature set indicates the yield percentage that was measured when the particular therapeutic protein was purified in the chromatography process. In some embodiments, all features and labels are numerical, with non-numerical classifications or categories being mapped to numerical values (e.g., with the allowable values [Monoclonal, Bispecific Format 1, Bispecific Format 2, Bispecific Format 1 or 2] of a modality feature/input being mapped to the values [00, 10, 01, 11]).

In some embodiments, training server 104 uses additional labeled data sets in training database 140 in order to validate the trained ML model(s) 108 (e.g., to confirm that a given one of ML model(s) 108 provides at least some minimum acceptable accuracy). In some embodiments, training server 104 also updates/refines one or more of ML model(s) 108 on an ongoing basis. For example, after ML model(s) 108 is/are initially trained to provide a sufficient level of accuracy, additional measurements of chromatography process performance indicators (and corresponding input/features) may be used to improve prediction accuracy.

Application 130 may retrieve, from training server 104 via network 106 and network interface 122, a specific one of ML model(s) 108 that corresponds to a performance indicator of interest. The performance indicator may be one that was indicated by a user via a GUI that was generated or populated by visualization unit 136, for example. Upon retrieving the model, computing system 102 stores a local copy as local ML model 138. In other embodiments, as noted above, no model is retrieved, and input/feature data is instead sent to training server 104 (or another server) as needed to use the appropriate model of model(s) 108, or all of model(s) 108 may reside only at computing system 102.

The performance indicator that a particular model 108 or model 138 is trained to predict may include an indicator of any aspect of performance, such as yield or product quality (e.g., purity). Moreover, the performance indicators may be generic to different types of chromatography (e.g., yield), or may be specific to a particular type of chromatography (e.g., CEX, SEC, Protein A, etc.). For example and without limitation, an ML model 108 or 138 may predict any of the following indicators: step yield, CEX % Acidic, CEX % Main, CEX % Basic, SEC % high molecular weight (HMW), SEC % Main, SEC % low molecular weight (LMW), capillary electrophoresis sodium dodecyl sulfate with reduced sample preparation (rCE-SDS) % Main, rCE-SDS % LMW, rCE-SDS % light chain (LC) + heavy chain (HC), capillary electrophoresis sodium dodecyl sulfate without reduced sample preparation (nrCE-SDS) % Main, rCE-SDS % Pre-LC, rCE-SDS % LC, rCE-SDS % non-glycosylated heavy chain (NGHC), rCE-SDS % HC, rCE-SDS % HMW, rCE-SDS % Pre-LC+LC+HC, pool conductivity (mS/cm), capillary isoelectric focusing (clEF) % Acidic, clEF % Basic, clEF % Main, nrCE-SDS % Pre-Peak, host cell protein (HCP), and SE-HPLC HMW. In some embodiments, the process parameters that a particular model 108 or model 138 is trained to predict may include an indicator of any aspect of the process, or an accuracy range thereof (for example, a confidence interval such as an 80%, 85%, 90%, or 95% confidence interval). For example and without limitation, an ML model 108 or 138 may predict any of the following process parameters: buffer and/or elution buffer and/or load pH, elution buffer molarity, elution buffer conductivity, gradient slope, linear velocity, load conductivity, load factor, stop collect, column volume, actual CEX loading (if CEX is used), loading flow rate, elution flow rate, buffer concentration, gradient length, gradient start, gradient end, pool volume, protein concentration, pool start, and/or pool end.

In accordance with the feature set used by model 138, data collection unit 132 collects the necessary data. For example, data collection unit 132 may receive user-entered process parameters (or performance indicators, as applicable), as well as molecular descriptors output by MOE application 139 for the therapeutic protein of interest (e.g., after the user enters or otherwise provides the amino acid sequence of the protein to the MOE application 139). The process parameters and performance indicators may be as described herein. For example, the process parameters may include any parameters relating to the conditions or characteristics of the hypothetical chromatography process, such as, for example and without limitation: buffer pH, elution buffer conductivity (mS/cm), elution buffer molarity (mM), elution buffer pH, gradient slope (mM/CV), linear velocity (cm/hr), load conductivity (mS/cm), load factor (g/Lr), load pH, stop collect (%), column volume, actual CEX loading, loading flow rate, elution flow rate, buffer concentration, gradient length, gradient start, gradient end, pool volume, protein concentration, pool start, and/or pool end.

The molecular descriptors generated by MOE application 139 may include any suitable descriptor types, such as, for example and without limitation, any or all of the following descriptors known to users of MOE software: pH, HI, pro_Fv_net_charge, U, asa_hyd, viscosity, hyd_idx, pro_helicity, apol, asa_hph, pro_net_charge, hyd_idx_cdr, pro_henry, b_1rotR, volume, amphipathicity, hyd_strength, pro_hyd_moment, b_rotR, mobility, ASPmax, hyd_strength_cdr, pro_mass, density, helicity, BSA, Packing Score, pro_mobility, ens_dipole, henry, BSA_HC, pro_affinity, pro_pI_3D, mass, net_charge, BSA_LC_HC, pro_app_charge, pro_pI_seq, pI_seq, app_charge, contact energy, pro_asa_hph, pro_r_gyr, pI_3D, dipole_moment, DRT, pro_asa_hyd, pro_r_solv, coeff_fric, hyd_moment, E bond, pro_asa_vdw, pro_sed_const, coeff_diff, zeta, E ele, pro_cdr_net_charge, pro_stability, r_gyr, zdipole, E sol, pro_coeff_diff, pro_volume, r_solv, zquadrupole, E vdw, pro_coeff_fric, pro_zdipole, sed_const, Eint_VL_VH, pro_dipole_moment, pro_zeta, eccen, GB/VI, pro_eccen, pro_zquadrupole, and/or asa_vdw. Preferably, in some embodiments, the molecular descriptors include at least one descriptor that is a function of (or otherwise dependent on) the pH level of the molecule's environment, and possibly many such descriptors. For example, various descriptors may depend on the surface charge of the protein molecule, and the surface charge may in turn depend on the pH of the molecule environment.

After data collection unit 132 has collected the process parameters (or performance indicators, as applicable) and molecular descriptors for a particular hypothetical chromatography process (possibly along with other data, such as a user-entered modality of the protein), prediction unit 134 causes ML model 138 to operate on those inputs/features to predict the desired performance indicator (or process parameter) for the hypothetical chromatography process. It is understood that, in some embodiments and/or scenarios, prediction unit 134 may obtain multiple, different local ML models 138 from training server 104 in order to predict (e.g., in parallel or sequentially) different performance indicators (or process parameters, as applicable) for the same hypothetical chromatography process, with the local ML models 138 operating on the same or different features to generate the respective predictions.

Visualization unit 136 causes a GUI, depicted on display 124, to present the predicted performance indicator(s) (or process parameters, as applicable), and/or other information derived from the predicted performance indicator(s) (or process parameters, as applicable). For example, visualization unit 138 may cause the GUI to present an indication of whether the predicted performance indicator(s) satisfy one or more acceptability criteria (e.g., after application 130 compares the performance indicator(s) to one or more respective threshold values). For example, visualization unit 138 may cause the GUI to present an accuracy range of the predicted process parameters.

The above prediction/visualization process may be repeated for a number of different hypothetical chromatography processes (e.g., for different combinations of process parameters with a fixed set of molecular descriptors for the therapeutic protein), thereby enabling a user to quickly test different process designs. A user can also quickly test particular aspects of a design, such as how small perturbations to a specific input (e.g., reflecting expected ranges in elution buffer pH, or loading flow rate, etc.) are likely to affect the predicted performance indicator(s). Visualization unit 136 may generate or populate one or more GUIs that help a viewing user to comprehend and consider the results of the predictions for the various hypothetical chromatography processes. In this manner, the viewing user(s) may make an informed selection of which chromatography process parameters to use in a real-world, commercial manufacturing process (subject to any necessary qualification testing). The selection of chromatography process parameters should generally attempt to maximize yield while minimizing impurities (possibly weighing one of these goals more than the other depending on the use case/project goals), and may be decided by one or more users based on the displayed information, or may be fully automated according to some predetermined selection criteria. In some cases, the techniques described herein may be used not only to select chromatography process parameters but also, or instead, to provide holistic insights into interrelations between new molecules and the purification process (e.g., by tweaking the molecular descriptors and observing the effect on various performance indicators). These insights can help guide molecular design in the future, by identifying key molecular characteristics that affect purification effectiveness.

To avoid the time and cost of having to perform and collect a very large amount of labeled historical data, interpretable machine learning models may be used as model(s) 108. For example, training server 104 may train one of model(s) 108 on hundreds of features, after which training server 104 (or a human reviewer) may analyze the trained model (e.g., weights assigned to each feature) to determine the most predictive features (e.g., about 10 features, or about 50 features, etc.). Thereafter, that particular model 108, or a new version of that model 108 that has been trained using only the most predictive features, may be used with a much smaller feature set. Identifying highly predictive features may also be useful for other purposes, such as providing new scientific insights that may give rise to new hypotheses, which could in turn lead to bioprocess improvements.

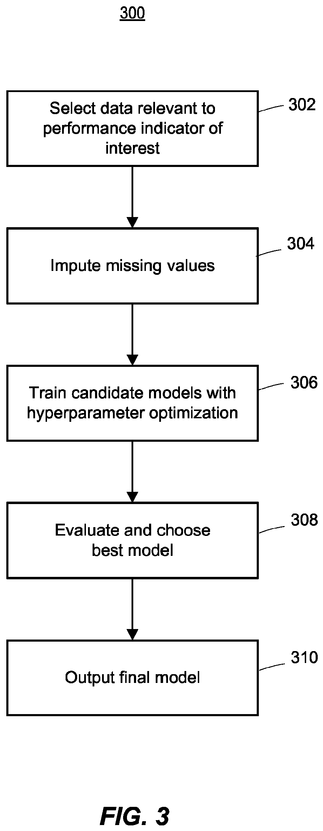

Various techniques for determining which models are best suited for particular performance indicators (and/or process parameters), and for identifying the most predictive features for a given model or use case, are now described with reference to FIGs. 3 and 4.

Generally, well-performing models for specific performance indicators may be identified by training a number of diverse model types using real-world, historical training data from previous chromatography purification processes, and comparing the results. FIG. 3 depicts an example process 300 that may be used to this end, for a particular performance indicator of interest. At a first stage 302 of the process 300, data that is relevant to the performance indicator is selected (i.e., identified and obtained). However, historical data can often be inconsistent, e.g., with different types and/or formats of data being captured for different drug products or projects. Thus, at stage 304, it may be necessary to impute missing values, and/or take other steps to ensure a robust set of training data (e.g., normalizing, removing outliers, etc.).

At stage 306, each of the candidate models is trained on at least a portion of the historical data, with hyperparameters being optimized for each candidate model. Stage 306 may include performing k-fold validation for each model (e.g., with k = 10, where a model is trained and evaluated 10 times across different 90/10 partitions of the dataset that was selected at stage 302 and augmented at stage 304, or with k = 5, etc.). Stage 306 may include tuning the hyperparameters of each model using a Bayesian search technique. The Bayesian technique performs a Bayesian-guided search that is computationally more efficient than a grid search or a random search, yet yields similar levels of performance as a random search. Stage 306 may include a number of iterations of Bayesian search, and choosing the model hyperparameters through k-fold validation.

At stage 308, the various candidate models (with their tuned hyperparameters) are evaluated, and a best model (for the performance indicator or process parameter of interest) is chosen. Any suitable criteria may be used to select a "best" model. For example, algorithm performance metrics such as the coefficient of determination ($R^2$) and/or root mean squared error (RMSE) may be captured for each model, with an average for each being obtained based on the cross-validation process. $R^2$ may be calculated as:

$$R^2 = 1 - \frac{\sum_{i=1}^{n}(\widehat{f_i} - y_i)^2}{\sum_{i=1}^{n}(\bar{y} - y_i)^2},$$
(Equation 1)

In Equation 1, n represents the number of samples per cross-validation fold, y represents the true target output, and $f$ represents the output predicted by the model. Average $R^2$ may be calculated as:

$$Avg\ R^2 \equiv \frac{1}{k}\sum_{j=1}^{k} R^2{}_j,$$
(Equation 2)

where k represents the number of cross-validation folds. RMSE may be calculated as:

$$RMSE \equiv \sqrt{\frac{1}{n}\sum_{i=1}^{n}(\widehat{f_i} - y_i)^2}$$
(Equation 3)

Average RMSE for a model may be calculated as:

$$RMSE_{avg} = \frac{1}{k}\sum_{j=1}^{k} RMSE_j$$
(Equation 4)

RMSE may be a better metric than $R^2$, because RMSE indicates the model accuracy/error in the easily-understood units of the performance indicator (or process parameter) being predicted. Furthermore, the $R^2$ metric can occasionally yield extremely negative values with some cross-validation sets, which can skew the model comparison when averaged across sets. RMSE may also be preferable to mean absolute error (MAE), because the former penalizes larger errors between the predictions and the actual results.

Thereafter, at stage 310, a final model for predicting the performance indicator (or process parameter) is output/selected, e.g., based on a comparison of RMSE (and/or one or more other metrics) for the different models. The final model may be re-trained on the entire dataset. The final production model is then stored as a trained model (e.g., one of ML model(s) 108), and is ready to make predictions for new/future chromatography processes.

In one embodiment, process 300 is performed by training server 104 of FIG. 1 (possibly with human input at various stages, such as selecting performance indicators of interest, selecting models as candidates, etc.). Process 300 may repeated for each performance indicator of interest, and for any suitable number of performance indicators (e.g., 5, 10, 20, etc.), such as any of the example performance indicators discussed elsewhere herein. As final models for the different performance indicators are output at each iteration of stage 310, training server 104 may add those final models to ML models 108. Thereafter, and prior to making a prediction for a particular, hypothetical chromatography purification process in the manner discussed herein (e.g., with reference to FIG. 1), computing system 102 or training server 104 may select the appropriate final model from ML models 108. The selection may be made based on user input indicating the desired performance indicator, for example.

Generally following the process 300, a number of models have been identified as having superior performance with respect to various different performance indicators for a chromatographic purification process during manufacture of a therapeutic protein, based on RMSE and as shown in Table 1 below:

TABLE 1

| Performance Indicator | Model with lowest Average RMSE | RMSE | RMSE / (RMSE range size) | Total # of Observations |
|---|---|---|---|---|
| rCE-SDS % LC + HC | Elastic Net | 0.282 | 28% | 41 |
| rCE-SDS % LC | Elastic Net | 0.170 | 28% | 71 |
| nrCE-SDS % Main | Regression Tree | 0.399 | 17% | 160 |
| rCE-SDS % Pre-LC | Regression Tree | 0.044 | 44% | 71 |
| rCE-SDS % NGHC | Regression Tree | 0.059 | 12% | 71 |
| rCE-SDS % HC | Regression Tree | 0.272 | 25% | 71 |
| rCE-SDS % HMW | Regression Tree | 0.213 | 35% | 71 |
| rCE-SDS % Pre-LC + LC + HC | Regression Tree | 0.141 | 16% | 71 |
| Pool Conductivity (mS/cm) | Regression Tree | 0.442 | 7% | 55 |
| nrCE-SDS % Pre-Peak | Regression Tree | 0.340 | 20% | 82 |
| CEX % Basic | Regression Tree | 0.741 | 2% | 492 |
| SEC % HMW | Regression Tree | 0.247 | 10% | 811 |
| SEC % Main | Regression Tree | 0.225 | 10% | 541 |
| SEC % LMW | Regression Tree | 0.035 | 3% | 339 |
| CEX % Acidic | XGBoost | 0.763 | 3% | 446 |
| cIEF % Acidic | XGBoost | 1.203 | 17% | 90 |
| CEX % Main | XGBoost | 0.845 | 2% | 543 |
| cIEF % Basic | XGBoost | 0.725 | 17% | 90 |
| cIEF % Main | XGBoost | 1.214 | 18% | 90 |
| Step Yield (%) | XGBoost | 3.003 | 5% | 879 |
| rCE-SDS % Main | XGBoost | 0.483 | 18% | 86 |
| rCE-SDS % LMW | XGBoost | 0.239 | 7% | 157 |

The results of Table 1 all pertain to historical datasets for various monoclonal antibodies. For each performance indicator, the model that yielded the lowest RMSE was interpreted to be the "best performing" model. As seen in Table 1, no one model performed best for predicting all performance indicators. Rather, a regression tree model performed best for 12 performance indicators, an xgboost model performed best for eight performance indicators, and elastic net performed best for two performance indicators. Other models (lasso, ridge, SGD, linear SVM, and PLS) evaluated with the process 300 did not perform the best for any of the performance indicators in Table 1. Regression tree and xgboost models, in particular, performed well with both large and small numbers of observations (training datasets).

Other processes may have different performance characteristics, due to larger or smaller training datasets, evaluating models based on different performance indicators (and/or based on combinations of different performance indicators), evaluating models using different metrics (other than RMSE), and so on. For example, when evaluating machine learning models for predicting yield and models for predicting SEC-HMW percentages (specifically for monoclonal antibodies) according to the $R^2$ metric, the best performing model was found to be the xgboost model. For this latter evaluation, process parameter inputs to the xgboost model included column volume, load pH, actual CEX loading, loading flow rate, elution flow rate, buffer concentration, elution pH, gradient slope, gradient length, gradient start, gradient end, pool volume, protein concentration, pool start, and/or pool end.

As noted above, it may be advantageous to learn which features are most important for a particular model so that, when the "best" model is identified/output at stage 310, only those features that are most predictive of the desired performance indicator are utilized. FIGs. 4A and 4B depict plots 400, 420 of example feature importance metrics (relative feature importance and feature correlation, respectively), both for predicting experimental yield and for predicting SE-HPLC HMW, using an eXtreme gradient boost (xgboost) model. The plots 400, 420 may be generated by visualization unit 136 and presented on a GUI via display 124, for example.

Plots such as the plots 400, 420 may allow users (e.g., scientists) to easily identify the most significant factors for predicting specific performance indicators (here, to predicting experimental yield and SE-HPLC HMW). This can also provide greater insight into how the structure of a molecule affects the purification process. For example, while it is common knowledge that hydrophobicity plays a role in increasing impurity in a CEX process, it is generally believed that the charge of the molecule would have the greatest effect. However, feature importance plots similar to plots 400 and/or 420 (or correlation heat maps, etc.) consistently and surprisingly rank hydrophobicity as a more significant indicator of higher impurities (specifically, high HMW) following a CEX process. Additionally, not all forms of hydrophobicity affect the level of impurity equally. For example, plots similar to plots 420 (or correlation heat maps, etc.) show that some forms of hydrophobicity are indicative of lower impurities (specifically, less HMW), at least relative to other hydrophobicities.

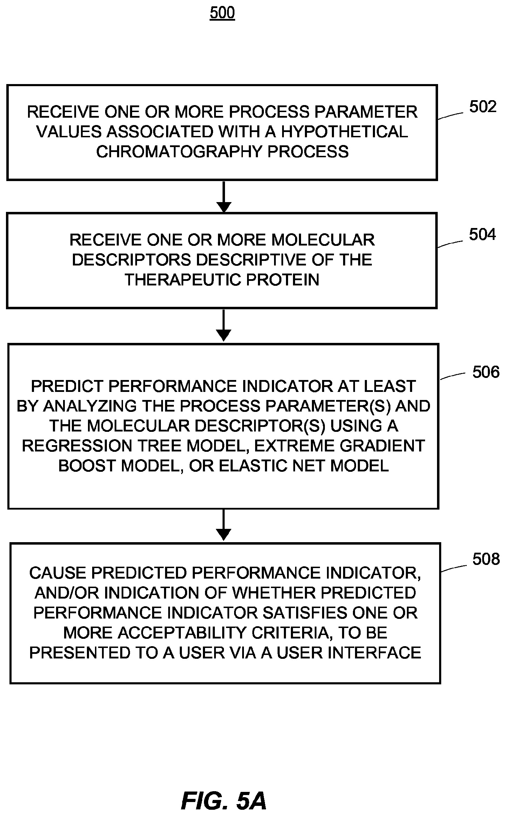

FIG. 5A is a flow diagram of an example method 500 for facilitating selection of chromatography parameters for a purification process during the manufacture of therapeutic proteins. The method 500 may be implemented, at least in part, by processing unit 120 of computing system 102 when executing the software instructions of application 130 stored in memory unit 128, or by one or more processors of server 104 (e.g., in a cloud service implementation), for example.

At block 502, one or more process parameter values associated with a hypothetical chromatography (e.g., CEX, SEC, or Protein A chromatography) process are received. The process parameter value(s) may be received via a user interface, for example, and/or by importing a file or other data, etc. As examples, and without limitation, the process parameter value(s) may include values of one or more of any of the following: buffer pH, elution buffer conductivity (mS/cm), elution buffer molarity (mM), elution buffer pH, gradient slope (mM/CV), linear velocity (cm/hr), load conductivity (mS/cm), load factor (g/Lr), load pH, stop collect (%), column volume, actual CEX loading, loading flow rate, elution flow rate, buffer concentration, gradient length, gradient start, gradient end, pool volume, protein concentration, pool start, and/or pool end. While example units are shown in the preceding list, it will be appreciated that these units are for illustrative purposes only, and that these parameters may be conveyed in any suitable units. Accordingly, it will be appreciated that the example units may be omitted from the preceding list, or any other list of process parameters herein.

At block 504, one or more molecular descriptors that are descriptive of the therapeutic protein are received. The molecular descriptor(s) may be received via a user interface, for example, and/or by importing a file or other data (e.g., from MOE application 139), etc. In some embodiments, method 500 also includes determining one or more molecular descriptors based on sequence information associated with the therapeutic protein (e.g., amino acid sequence information entered into MOE software), and/or based on an experimental measurement of a physical characteristic of the therapeutic protein (e.g., a measurement result entered into MOE software). In some embodiments, at least one molecular descriptor is a function of pH of the environment surrounding the molecule (e.g., a mathematical function that varies when a pH is known/specified). As examples, and without limitation, the molecular descriptor(s) may include any one or more of the following: pH, HI, pro_Fv_net_charge, U, asa_hyd, viscosity, hyd_idx, pro_helicity, apol, asa_hph, pro_net_charge, hyd_idx_cdr, pro_henry, b_1rotR, volume, amphipathicity, hyd_strength, pro_hyd_moment, b_rotR, mobility, ASPmax, hyd_strength_cdr, pro_mass, density, helicity, BSA, Packing Score, pro_mobility, ens_dipole, henry, BSA_HC, pro_affinity, pro_pl_3D, mass, net_charge, BSA_LC_HC, pro_app_charge, pro_pl_seq, pl_seq, app_charge, contact energy, pro_asa_hph, pro_r_gyr, pl_3D, dipole_moment, DRT, pro_asa_hyd, pro_r_solv, coeff_fric, hyd_moment, E bond, pro_asa_vdw, pro_sed_const, coeff_diff, zeta, E ele, pro_cdr_net_charge, pro_stability, r_gyr, zdipole, E sol, pro_coeff_diff, pro_volume, r_solv, zquadrupole, E vdw, pro_coeff_fric, pro_zdipole, sed_const, Eint_VL_VH, pro_dipole_moment, pro_zeta, eccen, GB/VI, pro_eccen, pro_zquadrupole, and/or asa_vdw.

At block 506, a performance indicator for the hypothetical chromatography process is predicted, at least by analyzing the process parameter(s) received at block 502, and the molecular descriptor(s) received at block 504, using a machine learning model. The machine learning model may be either a regression tree model, an eXtreme gradient boost (xgboost) model, or an elastic net model. As examples, and without limitation, the predicted performance indicator may be one of the following: Step Yield, CEX % Acidic, CEX % Main, CEX % Basic, SEC % HMW, SEC % Main, SEC % low molecular weight (LMW), capillary electrophoresis sodium dodecyl sulfate with reduced sample preparation (rCE-SDS) % Main, rCE-SDS % LMW, rCE-SDS % light chain (LC) + heavy chain (HC), capillary electrophoresis sodium dodecyl sulfate without reduced sample preparation (nrCE-SDS) % Main, rCE-SDS % Pre-LC, rCE-SDS % LC, rCE-SDS % non-glycosylated heavy chain (NGHC), rCE-SDS % HC, rCE-SDS % high molecular weight (HMW), rCE-SDS % Pre-LC+LC+HC, pool conductivity (mS/cm), capillary isoelectric focusing (clEF) % Acidic, clEF % Basic, clEF % Main, nrCE-SDS % Pre-Peak, HCP, and/or SE-HPLC HMW.

In some embodiments, block 506 includes using a regression tree model to predict nrCE-SDS % LC + HC, rCE-SDS % Pre-LC, CEX % Basic, SEC % HMW, SEC % Main, SEC % LMW, rCE-SDS % HC, rCE-SDS % HMW, rCE-SDS % Pre-LC+LC_HC, pool conductivity, or nrCE-SDS % Pre-Peak. In other embodiments, block 506 includes using an eXtreme gradient boost model to predict CEX % Acidic, CEX % Main, step yield, rCE-SDS % Main, rCE-SDS % LMW, clEF % Acidic, clEF % Basic, or clEF % Main. Alternatively, block 506 may include using an eXtreme gradient boost model to predict SEC % HMW or yield. In still other embodiments, block 506 includes using an elastic net model to predict rCE-SDS % LC + HC, or to predict rCE-SDS % LC.

At block 508, the performance indicator predicted at block 506, and/or an indication of whether the predicted performance indicator satisfies one or more acceptability criteria (e.g., exceeds, or is below, some threshold value), is caused to be presented to a user via a user interface (e.g., a GUI generated or populated by visualization unit 136 and presented on display 124 of FIG. 1), to facilitate the selection (e.g., manual selection by a user) of chromatography parameters for a real-world purification process during the manufacture of the therapeutic protein.

In some embodiments, the method 500 includes one or more additional blocks not shown in FIG. 5A. For example, method 500 may include two additional blocks that both occur prior to block 502: a first additional block in which data indicative of a performance indicator of interest is received from a user via a user interface (e.g., a GUI generated or populated by visualization unit 136 and presented on display 124), and a second additional block in which the machine learning model (later used at block 506) is selected from among multiple machine learning models (e.g., ML models 108) that were trained to predict different performance indicators.

As another example, method 500 may include four additional blocks, similar to blocks 506 and 508 (or 502 through 508), to occur for a second performance indicator of interest, using a second machine learning model. For example, the first and second machine learning models may be xgboost models that were trained for a different purpose, with one predicting experimental yield percentage and the other predicting SEC HMW percentage.

As yet another example, method 500 may include two additional blocks that both occur after block 506: a first additional block in which one or more process parameter values are selected for a (real-world) chromatography process for the therapeutic protein based on the performance indicator and/or the indication presented at block 508, and a second additional block in which the chromatography process is performed for the therapeutic protein according to the one or more selected process parameter values.

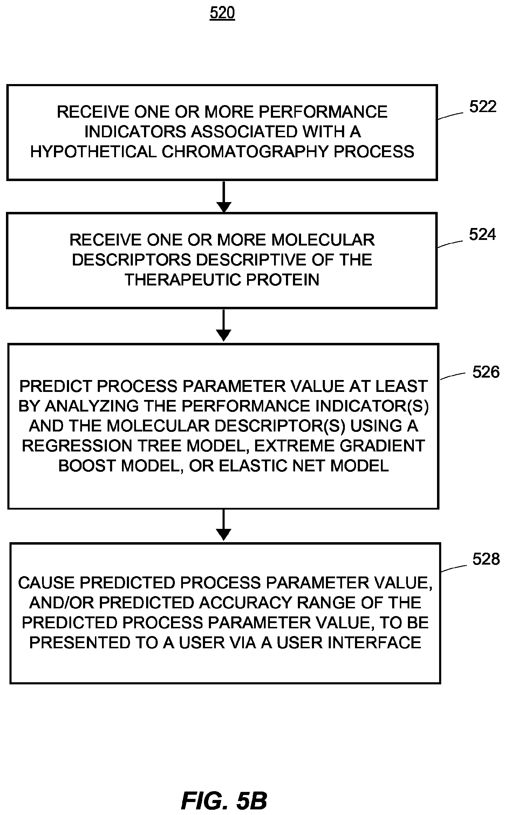

FIG. 5B is a flow diagram of another example method 520 for facilitating selection of chromatography parameters for a purification process during the manufacture of therapeutic proteins. The method 520 may be implemented, at least in part, by processing unit 120 of computing system 102 when executing the software instructions of application 130 stored in memory unit 128, or by one or more processors of server 104 (e.g., in a cloud service implementation), for example.

At block 522, one or more performance indicators associated with a hypothetical chromatography (e.g., CEX, SEC, or Protein A chromatography) process are received. The performance indicator(s) may be received via a user interface, for example, and/or by importing a file or other data, etc. As examples, and without limitation, the performance indicator(s) may include values of one or more of any of the following: Step Yield, CEX % Acidic, CEX % Main, CEX % Basic, SEC % HMW, SEC % Main, SEC % low molecular weight (LMW), capillary electrophoresis sodium dodecyl sulfate with reduced sample preparation (rCE-SDS) % Main, rCE-SDS % LMW, rCE-SDS % light chain (LC) + heavy chain (HC), capillary electrophoresis sodium dodecyl sulfate without reduced sample preparation (nrCE-SDS) % Main, rCE-SDS % Pre-LC, rCE-SDS % LC, rCE-SDS % non-glycosylated heavy chain (NGHC), rCE-SDS % HC, rCE-SDS % high molecular weight (HMW), rCE-SDS % Pre-LC+LC+HC, pool conductivity (mS/cm), capillary isoelectric focusing (clEF) % Acidic, clEF % Basic, clEF % Main, nrCE-SDS % Pre-Peak, HCP, and/or SE-HPLC HMW.

At block 524, one or more molecular descriptors that are descriptive of the therapeutic protein are received. The molecular descriptor(s) may be received via a user interface, for example, and/or by importing a file or other data (e.g., from MOE application 139), etc. In some embodiments, method 520 also includes determining one or more molecular descriptors based on sequence information associated with the therapeutic protein (e.g., amino acid sequence information entered into MOE software), and/or based on an experimental measurement of a physical characteristic of the therapeutic protein (e.g., a measurement result entered into MOE software). In some embodiments, at least one molecular descriptor is a function of pH of the environment surrounding the molecule (e.g., a mathematical function that varies when a pH is known/specified). As examples, and without limitation, the molecular descriptor(s) may include any one or more of the following: pH, HI, pro_Fv_net_charge, U, asa_hyd, viscosity, hyd_idx, pro_helicity, apol, asa_hph, pro_net_charge, hyd_idx_cdr, pro_henry, b_1rotR, volume, amphipathicity, hyd_strength, pro_hyd_moment, b_rotR, mobility, ASPmax, hyd_strength_cdr, pro_mass, density, helicity, BSA, Packing Score, pro_mobility, ens_dipole, henry, BSA_HC, pro_affinity, pro_pI_3D, mass, net_charge, BSA_LC_HC, pro_app_charge, pro_pI_seq, pI_seq, app_charge, contact energy, pro_asa_hph, pro_r_gyr, pI_3D, dipole_moment, DRT, pro_asa_hyd, pro_r_solv, coeff_fric, hyd_moment, E bond, pro_asa_vdw, pro_sed_const, coeff_diff, zeta, E ele, pro_cdr_net_charge, pro_stability, r_gyr, zdipole, E sol, pro_coeff_diff, pro_volume, r_solv, zquadrupole, E vdw, pro_coeff_fric, pro_zdipole, sed_const, Eint_VL_VH, pro_dipole_moment, pro_zeta, eccen, GB/VI, pro_eccen, pro_zquadrupole, and/or asa_vdw.

At block 526, a process parameter value for the hypothetical chromatography process is predicted, at least by analyzing the performance indicator(s) received at block 522, and the molecular descriptor(s) received at block 524, using a machine learning model. The machine learning model may be either a regression tree model, an eXtreme gradient boost (xgboost) model, or an elastic net model. As examples, and without limitation, the process parameter for which a value is predicted may be one of the following: buffer pH, elution buffer conductivity (mS/cm), elution buffer molarity (mM), elution buffer pH, gradient slope (mM/CV), linear velocity (cm/hr), load conductivity (mS/cm), load factor (g/Lr), load pH, stop collect (%), column volume, actual CEX loading, loading flow rate, elution flow rate, buffer concentration, gradient length, gradient start, gradient end, pool volume, protein concentration, pool start, and/or pool end. While example units are shown in the preceding list, it will be appreciated that these units are for illustrative purposes only, and that these parameters may be conveyed in any suitable units. Accordingly, it will be appreciated that the example units may be omitted from the preceding list, or any other list of process parameters herein.

In some embodiments, block 526 includes using a regression tree model to predict the process parameter value based on the molecular descriptor(s) and one or more of nrCE-SDS % LC + HC, rCE-SDS % Pre-LC, CEX % Basic, SEC % HMW, SEC % Main, SEC % LMW, rCE-SDS % HC, rCE-SDS % HMW, rCE-SDS % Pre-LC+LC_HC, pool conductivity, and/or nrCE-SDS % Pre-Peak. In other embodiments, block 526 includes using an eXtreme gradient boost model to predict the process parameter value based on the molecular descriptor(s) and one or more of CEX % Acidic, CEX % Main, step yield, rCE-SDS % Main, rCE-SDS % LMW, clEF % Acidic, clEF % Basic, and/or clEF % Main. Alternatively, block 506 may include using an eXtreme gradient boost model to predict the process parameter value based on the molecular descriptor(s) and one or both of SEC % HMW and yield. In still other embodiments, block 506 includes using an elastic net model to predict the process parameter value based on the molecular descriptor(s) and rCE-SDS % LC + HC, or based on the molecular descriptor(s) and rCE-SDS % LC.

At block 528, the process parameter value predicted at block 526, and/or a predicted accuracy range of the predicted process parameter value, is caused to be presented to a user via a user interface (e.g., a GUI generated or populated by visualization unit 136 and presented on display 124 of FIG. 1), to facilitate the selection (e.g., manual selection by a user) of chromatography parameters for a real-world purification process during the manufacture of the therapeutic protein.

In some embodiments, the method 520 includes one or more additional blocks not shown in FIG. 5B. For example, method 520 may include two additional blocks that both occur prior to block 522: a first additional block in which data indicative of a process parameter of interest is received from a user via a user interface (e.g., a GUI generated or populated by visualization unit 136 and presented on display 124), and a second additional block in which the machine learning model (later used at block 526) is selected from among multiple machine learning models (e.g., ML models 108) that were trained to predict values of different process parameters.

As another example, method 520 may include four additional blocks, similar to blocks 526 and 528 (or 522 through 528), to occur for a second process parameter of interest, using a second machine learning model. For example, the first and second machine learning models may be xgboost models that were trained for a different purpose, with one predicting buffer pH and the other predicting load factor.

As yet another example, method 520 may include two additional blocks that both occur after block 526: a first additional block in which one or more process parameter values are selected for a (real-world) chromatography process for the therapeutic protein based on the information presented at block 528, and a second additional block in which the chromatography process is performed for the therapeutic protein according to the one or more selected process parameter values.

Although the systems, methods, devices, and components thereof, have been described in terms of exemplary embodiments, they are not limited thereto. The detailed description is to be construed as exemplary only and does not describe every possible embodiment of the invention because describing every possible embodiment would be impractical, if not impossible. Numerous alternative embodiments could be implemented, using either current technology or technology developed after the filing date of this patent that would still fall within the scope of the claims defining the invention.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

Embodiments

Preferred embodiments of the invention are as follows:

    1. A method for facilitating selection of chromatography parameters for a purification process during manufacture of a therapeutic protein, the method comprising:

receiving, by one or more processors of a computing system, one or more process parameter values associated with a hypothetical chromatography process;

receiving, by the one or more processors, one or more molecular descriptors descriptive of the therapeutic protein;

predicting, by the one or more processors, a performance indicator for the hypothetical chromatography process at least by analyzing the one or more process parameters and the one or more molecular descriptors using a machine learning model, wherein the machine learning model is a model selected from a group consisting of (i) a regression tree model, (ii) an eXtreme gradient boost model, and (iii) an elastic net model; and

causing, by the one or more processors, one or both of (i) the predicted performance indicator, and (ii) an indication of whether the predicted performance indicator satisfies one or more acceptability criteria, to be presented to a user via a user interface.

2. A method for facilitating selection of chromatography parameters for a purification process during manufacture of a therapeutic protein, the method comprising:

receiving, by one or more processors of a computing system, one or more performance indicators associated with a hypothetical chromatography process;

receiving, by the one or more processors, one or more molecular descriptors descriptive of the therapeutic protein;

predicting, by the one or more processors, a process parameter value for the hypothetical chromatography process at least by analyzing the one or more performance indicators and the one or more molecular descriptors using a machine learning model, wherein the machine learning model is a model selected from a group consisting of (i) a regression tree model, (ii) an eXtreme gradient boost model, and (iii) an elastic net model; and

causing, by the one or more processors, one or both of (i) the predicted process parameter value, and (ii) a predicted accuracy range of the predicted process parameter value, to be presented to a user via a user interface.

3. The method of embodiment 1 or 2, wherein the hypothetical chromatography process is a process selected from a group consisting of:

a hypothetical cation-exchange chromatography (CEX) process;

a hypothetical size-exclusion chromatography (SEC) process; and

a Protein A chromatography process.

4. The method of any one of embodiment 1 through 3, further comprising:
determining, by the one or more processors, at least one of the one or more molecular descriptors based on sequence information associated with the therapeutic protein.

5. The method of any one of embodiments 1 through 4, further comprising:
determining, by the one or more processors, at least one of the one or more molecular descriptors based on an experimental measurement of a physical characteristic of the therapeutic protein.

6. The method of any one of embodiments 1 through 5, wherein at least one of the one or more molecular descriptors is a function of pH level.

7. The method of any one of embodiments 1 through 6, wherein the one or more process parameter values include one or more of:

buffer pH;

elution buffer pH;

elution buffer conductivity;

elution buffer molarity;

gradient slope;

linear velocity;

load conductivity;

load factor;

load pH; or

stop collect.

8. The method of any one of embodiments 1 through 7, wherein the machine learning model is the regression tree model.

9. The method of embodiment 8, wherein the performance indicator includes:

nrCE-SDS % LC + HC;

rCE-SDS % Pre-LC;

CEX % Basic;

SEC % HMW;

SEC % Main;

SEC % LMW;

rCE-SDS % HC;

rCE-SDS % HMW;

rCE-SDS % Pre-LC+LC_HC;

pool conductivity; or

nrCE-SDS % Pre-Peak.

10. The method of any one of embodiments 1 through 7, wherein the machine learning model is the eXtreme gradient boost model.

11. The method of embodiment 10, wherein the performance indicator includes:

CEX % Acidic;

CEX % Main;

step yield;

rCE-SDS % Main;

rCE-SDS % LMW;

cIEF % Acidic;

cIEF % Basic; or

cIEF % Main.

12. The method of embodiment 9, wherein the performance indicator includes SEC % HMW.

13. The method of embodiment 12, further comprising:

predicting, by the one or more processors, a yield for the hypothetical chromatography process at least by analyzing process parameters and molecular descriptors using an additional machine learning model, wherein the additional machine learning model is another eXtreme gradient boost model; and

causing, by the one or more processors, one or both of (i) the predicted yield, and (ii) an indication of whether the predicted yield satisfies one or more additional acceptability criteria, to be presented to the user via the user interface.

14. The method of any one of embodiments 1 through 7, wherein the machine learning model is the elastic net model.

15. The method of embodiment 14, wherein the performance indicator includes:

rCE-SDS % LC + HC; or

rCE-SDS % LC.

16. The method of any one of embodiments 1 or 3 through 15, further comprising:

selecting one or more process parameter values for a chromatography process for the therapeutic protein based on the presented performance indicator and/or the presented indication; and

performing the chromatography process for the therapeutic protein according to the selected process parameter values.

17. The method of any one of embodiments 2 through 15, further comprising:

selecting one or more process parameter values for a chromatography process for the therapeutic protein based on the presented predicted process parameter value, and/or the predicted accuracy range; and

performing the chromatography process for the therapeutic protein according to the selected process parameter values.

18. One or more non-transitory, computer-readable media storing instructions that, when executed by one or more processors of a computing system, cause the computing system to perform the method of any one of embodiments 1 through 17.

19. A computing system comprising:

one or more processors; and

one or more non-transitory, computer-readable media storing instructions that, when executed by the one or more processors, cause the computing system to perform the method of any one of embodiments 1 through 15.

**Claims**

1.  A method for facilitating selection of chromatography parameters for a purification process during manufacture of a therapeutic protein, the method comprising:

receiving, by one or more processors of a computing system, one or more process parameter values associated with a hypothetical chromatography process, wherein the one or more process parameter values include one or more of: buffer pH; elution buffer pH; elution buffer conductivity; elution buffer molarity; gradient slope; linear velocity; load conductivity; load factor; load pH; or stop collect;

receiving, by the one or more processors, one or more molecular descriptors descriptive of the therapeutic protein;

predicting, by the one or more processors, a performance indicator for the hypothetical chromatography process, as an output of a machine learning model, at least by applying the one or more process parameter values and the one or more molecular descriptors as inputs to the machine learning model, wherein the machine learning model is an eXtreme gradient boost model for predicting a set of one or more performance indicators including:

cation-exchange chromatography percent Acidic (CEX % Acidic);
cation-exchange chromatography percent Main (CEX % Main);
step yield;
capillary electrophoresis sodium dodecyl sulfate with reduced sample preparation percent (rCE-SDS % Main);
capillary electrophoresis sodium dodecyl sulfate with reduced sample preparation percent (rCE-SDS % LMW);
capillary isoelectric focusing percent acidic (clEF % Acidic);
capillary isoelectric focusing percent basic (clEF % Basic); and/or
capillary isoelectric focusing percent main (clEF % Main); and

causing, by the one or more processors, one or both of (i) the predicted performance indicator, and (ii) an indication of whether the predicted performance indicator satisfies one or more acceptability criteria, to be presented to a user via a user interface.

2. The method of claim 1, wherein the hypothetical chromatography process is a process selected from a group consisting of:

a hypothetical cation-exchange chromatography (CEX) process;
a hypothetical size-exclusion chromatography (SEC) process; and
a Protein A chromatography process.

3. The method of claim 1 or 2, further comprising:
determining, by the one or more processors, at least one of the one or more molecular descriptors based on sequence information associated with the therapeutic protein.

4. The method of any one of claims 1 through 3, further comprising:
determining, by the one or more processors, at least one of the one or more molecular descriptors based on an experimental measurement of a physical characteristic of the therapeutic protein.

5. The method of any one of claims 1 through 4, wherein at least one of the one or more molecular descriptors is a function of pH level.

6. The method of any one of claims 1 through 5, wherein:

the performance indicator includes SEC % HMW; and
the method further comprises:

predicting, by the one or more processors, a yield for the hypothetical chromatography process at least by analyzing process parameters and molecular descriptors using an additional machine learning model, wherein the additional machine learning model is another eXtreme gradient boost model; and
causing, by the one or more processors, one or both of (i) the predicted yield, and (ii) an indication of whether the predicted yield satisfies one or more additional acceptability criteria, to be presented to the user via the user interface.

7. The method of any one of claims 1 through 6, further comprising:

selecting one or more process parameter values for a chromatography process for the therapeutic protein based on the presented performance indicator and/or the presented indication; and

performing the chromatography process for the therapeutic protein according to the selected process parameter values.

8. One or more non-transitory, computer-readable media storing instructions that, when executed by one or more processors of a computing system, cause the computing system to perform the method of any one of claims 1 through 6.

9. A computing system comprising:

one or more processors; and
one or more non-transitory, computer-readable media storing instructions that, when executed by the one or more processors, cause the computing system to perform the method of any one of claims 1 through 6.

FIG. 1

FIG. 2
(prior art)

Upstream Process

Downstream Process

| Centrifugation / Depth filtration | Chromatographic purification | Virus filtration | Concentration and formulation |

Drug Substance

EP 4 776 294 A2

<u>300</u>

```
┌─────────────────────────┐
│   Select data relevant to │ ── 302
│  performance indicator of │
│          interest         │
└─────────────────────────┘
            │
            ▼
┌─────────────────────────┐
│    Impute missing values  │ ── 304
└─────────────────────────┘
            │
            ▼
┌─────────────────────────┐
│  Train candidate models with │ ── 306
│  hyperparameter optimization │
└─────────────────────────┘
            │
            ▼
┌─────────────────────────┐
│     Evaluate and choose   │ ── 308
│         best model        │
└─────────────────────────┘
            │
            ▼
┌─────────────────────────┐
│     Output final model    │ ── 310
└─────────────────────────┘
```

*FIG. 3*

Feature Importance Plot: Experimental yield (%)

Feature Importance Plot: SE-HPLC HMW (%)

FIG. 4A

420

**Coefficient Plot:  Experimental yield (%)**

Feature Correlations

**Coefficient Plot:  SE-HPLC HMW (%)**

Feature Correlations

*FIG. 4B*

EP 4 776 294 A2

500

RECEIVE ONE OR MORE PROCESS PARAMETER VALUES ASSOCIATED WITH A HYPOTHETICAL CHROMATOGRAPHY PROCESS — 502

RECEIVE ONE OR MORE MOLECULAR DESCRIPTORS DESCRIPTIVE OF THE THERAPEUTIC PROTEIN — 504

PREDICT PERFORMANCE INDICATOR AT LEAST BY ANALYZING THE PROCESS PARAMETER(S) AND THE MOLECULAR DESCRIPTOR(S) USING A REGRESSION TREE MODEL, EXTREME GRADIENT BOOST MODEL, OR ELASTIC NET MODEL — 506

CAUSE PREDICTED PERFORMANCE INDICATOR, AND/OR INDICATION OF WHETHER PREDICTED PERFORMANCE INDICATOR SATISFIES ONE OR MORE ACCEPTABILITY CRITERIA, TO BE PRESENTED TO A USER VIA A USER INTERFACE — 508

*FIG. 5A*

520

RECEIVE ONE OR MORE PERFORMANCE INDICATORS ASSOCIATED WITH A HYPOTHETICAL CHROMATOGRAPHY PROCESS ⟋ 522

↓

RECEIVE ONE OR MORE MOLECULAR DESCRIPTORS DESCRIPTIVE OF THE THERAPEUTIC PROTEIN ⟋ 524

↓

PREDICT PROCESS PARAMETER VALUE AT LEAST BY ANALYZING THE PERFORMANCE INDICATOR(S) AND THE MOLECULAR DESCRIPTOR(S) USING A REGRESSION TREE MODEL, EXTREME GRADIENT BOOST MODEL, OR ELASTIC NET MODEL ⟋ 526

↓

CAUSE PREDICTED PROCESS PARAMETER VALUE, AND/OR PREDICTED ACCURACY RANGE OF THE PREDICTED PROCESS PARAMETER VALUE, TO BE PRESENTED TO A USER VIA A USER INTERFACE ⟋ 528

*FIG. 5B*